# EUROPÄISCHE PATENTANMELDUNG

(11) **EP 0 607 772 A2**
(43) Veröffentlichungstag der Anmeldung: **27.07.1994**
(21) Anmeldenummer: 94100007.7
(22) Anmeldetag: 03.01.1994
(51) Int. Cl.: C11D 1/62

(54) **Quaternierte Alkylaminalkoxylate als Viskositätsregulatoren für wässrige Dispersionen von stickstoffhaltigen kationenaktiven Wirkstoffkomponenten**

(30) Priorität: 16.01.1993 DE 4301073; 07.07.1993 DE 4322571
(71) Anmelder: HÜLS AKTIENGESELLSCHAFT, D-45764 Marl (DE)
(72) Erfinder: Beck, Rudolf, D-45770 Marl (DE); Gasber, Willi, D-45772 Marl (DE)

(57) **Zusammenfassung**

Die Erfindung betrifft die Verwendung von quaternierten Alkylaminalkoxylaten als Viskositätsregulatoren für wäßrige Dispersionen von stickstoffhaltigen kationenaktiven Wirkstoffkomponenten, die im wesentlichen in Wäscheweichspülerformul ierungen eingesetzt werden.

Durch den Zusatz der quaternierten Alkylaminalkoxylate kann die Lagerstabilität von bis zu 60 Gew.-%igen Fertigdispersionen so eingestellt werden, daß über einen Temperaturbereich von 0 bis 50 °C keine nachteilige Viskositätsveränderung, insbesondere kein störender Anstieg auftritt.

## Beschreibung

Die Erfindung betrifft die Verwendung von quaternierten Alkylaminalkoxylaten als Viskositätsregulatoren für wäßrige Dispersionen von stickstoffhaltigen kationenaktiven Wirkstoffkomponenten, die allgemein als Oberflächenbehandlungsmittel, z. B. zur Hydrophobierung von festen Oberflächen, insbesondere aber in Wäscheweichspülerformulierungen, eingesetzt werden.

In letzter Zeit haben zur Reduzierung des Distributionsaufwandes und zur Reduzierung der Verpackungsmittelmengen Oberflächenbehandlungsmittel mit erhöhtem Wirkstoffgehalt - sogenannte Konzentrate und insbesondere Konzentrate von Weichspülern - zunehmende Bedeutung gewonnen.

Die Problematik der Konzentrate besteht vor allem darin, daß deren Viskosität mit steigendem Gehalt an kationenaktiven Wirkstoffen exponentiell zunimmt.

Für den Einsatz z. B. konzentrierter Wäscheweichspülmittel sind jedoch insbesondere Lagerstabilität und niedrige Viskosität über einen möglichst breiten Temperaturbereich wichtige Voraussetzungen, weil sonst die kontrollierte Entnahme aus dem Vorratsgefäß erschwert und die entsprechende Dosierkammer im Waschautomaten nicht restlos ausgespült wird. Bei zu hoher Viskosität ist außerdem die Kaltwasserdispergierbarkeit nicht mehr gewährleistet, was eine ungenügende Verteilung in der Spülflotte und ein vermindertes und ungleichmäßiges Aufziehen auf die Faseroberfläche zur Folge hat. Auch Verdünnungen im Haushalt, bei denen aus sogenannten Nachfüllpacks das Konzentrat mit kaltem Wasser aufgefüllt wird, sind bei o. g. Produkteigenschaften nicht problemlos durchzuführen.

In der EP-A-0 498 050 werden Rohstoffe beschrieben, mit denen dünnflüssige lagerstabile Dispersionen von über 15 % Wirkstoffgehalt hergestellt werden können. Die Lagerstabilität bezieht sich dabei auf eine Lagerung bei Temperaturen von 20 und 40 °C.

Es wurde festgestellt, daß diese Lagerstabilität bei Temperaturen unterhalb von 20 °C nachläßt. So tritt bei kühler Lagerung eine deutlich sicht- und meßbare, nicht reversible Eindickung auf.

Zur Vermeidung dieses Nachteils wird die Zugabe von Polyglykolethern, Fettalkoholen (EP-A-0 013 780) bzw. Ethylenoxid-Addukten von Fettalkoholen (EP-A-0 280 550, EP-A-0 040 562, EP-A-0 507 478) als Viskositätsregulator empfohlen. Der Einfluß dieser Substanzen auf die Lagerstabilität ist jedoch im extremen Maße von der Zusammensetzung, insbesondere von Art und Gehalt des oder der Wirkstoffe sowie der Additive, abhängig. So treten Eindickungen insbesondere bei Wirkstoffkonzentraten mit mehr als 15 Gew.-% Aktivsubstanz auf. Es ist daher z. B. praktisch nicht möglich, stabile 20 Gew.-%ige Wirkstoffkonzentrate von Fettsäureestern der N-Alkyl-N,N,N-trihydroxyalkylammoniumsalze herzustellen.

Der Erfindung lag daher die Aufgabe zu Grunde, Wirkstoffkonzentrate von stickstoffhaltigen kationenaktiven Wirkstoffkomponenten mit Gehalten von über 15 Gew.-% zu entwickeln, die die genannten Nachteile nicht besitzen. Sie sollen insbesondere über einen größeren Temperaturbereich keine anwendungstechnisch nachteilige Viskositätsabhängigkeit zeigen und vor allem auch bei tiefen Temperaturen im Bereich von 0 bis 20 °C lagerstabil sein, d. h. sie dürfen auch nach mehreren Monaten keine Phasentrennung und kein Eindickverhalten zeigen.

Als stickstoffhaltige kationenaktive Wirkstoffkomponenten kommen insbesondere in Betracht:
- übliche Fettsäureester der N-Alkyl-N,N,N-trihydroxyalkylammoniumsalze, wie sie z. B. in der DE-OS 37 20 332 beschrieben werden,
- übliche quaternierte bzw. protonierte Imidazoline, wie z. B. 1-Methyl-2-Alkyl-3-alkylamido-ethylimidazolinium-methosulfat, wie sie z. B. von H. Baumung, L. Huber und D. Vetter in Seifen-ÖleFette-Wachse 117 (1991) 287 oder in EP 0 199 383 beschrieben werden,
- übliche 2,3-Dihydroxyalkyl-1-trialkylammoniumsalz-Derivate, wie sie z. B. von J. Waters et al. in Tenside Surf.Det. 28 (1991) 6 oder in DE 27 28 841 beschrieben werden,
- N,N-Di(alkyl)₁-N,N-di(alkyl)₂ammoniumsalze,
   wie sie z. B. in US 3 681 241 beschrieben werden,

- (alkyl)₁ =: unverzweigter oder verzweigter langkettiger Alkylrest mit 12 bis 20 Kohlenstoffatomen, insbesondere der Stearylrest
- (alkyl)₂ =: unverzweigter oder verzweigter kurzkettiger Alkylrest mit 1 bis 4 Kohlenstoffatomen, insbesondere der Methylrest

und all deren Mischungen.

Es wurde nun überraschend gefunden, daß bei Zusatz von hydrophilen quaternierten Alkylaminalkoxylaten, hergestellt durch Anlagerung von Ethylenoxid und/oder Propylenoxid an Alkylamine und anschließende Quaternierung, die unerwünschte Viskositätssteigerung der wäßrigen Dispersionen der stickstoffhaltigen kationenaktiven Wirkstoffkomponenten nicht mehr auftritt.

Gegenstand der Erfindung ist daher die Verwendung von quaternierten Alkylaminalkoxylaten der allgemeinen Formeln

R₁R₃N^{⊕}[(C₂H₄O)ₓ(C₃H₆O)_{y}-H]₂ X^{⊖} (I)

oder

R₁R₂R₃N^{⊕}[(C₂H₄O)ₓ(C₃H₆O)_{y}-H]X^{⊖} (II)

oder deren Mischungen,
wobei R₁ und R₂ unabhängig voneinander verzweigte oder geradkettige, gesättigte oder ungesättigte Alkylreste mit 8 bis 22, vorzugsweise 12 bis 18, Kohlenstoffatomen bedeuten, R₃ für einen Alkylrest mit 1 bis 4 Kohlenstoffatomen oder einen Alkylarylrest mit 7 bis 10 Kohlenstoffatomen steht, X^{⊖} einen einwertigen Salzrest darstellt und für x und y
x = 0 bis 150, vorzugsweise 10 bis 100,
y = 0 bis 100, vorzugsweise 0 bis 10 gilt und
x oder y mindestens 1 sein muß
und C₂H₄O eine Ethylenoxid-Moleküleinheit und C₃H₆O eine Propylenoxid-Moleküleinheit bedeutet,
als Viskositätsregulatoren für wäßrige Dispersionen von einzelnen stickstoffhaltigen kationenaktiven Wirkstoffkomponenten oder deren Mischungen und üblichen Zusatzstoffen.

Da es sich bei den Ethoxilierungsgraden x und Propoxilierungsgraden y um Mittelwerte handelt, können sie in den angegebenen Grenzen jeden beliebigen auch nicht ganzzahligen Wert annehmen.

Verwendet man beide Epoxide, so können diese entweder nacheinander oder gleichzeitig mit den Alkylaminen umgesetzt werden. Die Alkylenoxideinheiten können daher sowohl blockweise als auch statistisch verteilt eingebunden werden. Es kann sowohl die Ethylenoxid- als auch die Propylenoxideinheit dem Stickstoff benachbart sein.

Die quartären Ammonium-Verbindungen werden in üblicher Weise durch Peralkylierung der Mono- bzw. Dialkylaminalkoxylate mit kurzkettigen Alkylhalogeniden, Alkylarylhalogeniden oder Dialkylsulfaten hergestellt. Bevorzugt werden als Quaternierungsmittel Methylchlorid, Benzylchlorid oder Dimethylsulfat eingesetzt.

Die Quaternierung der Alkylaminalkoxylate erfolgt gewöhnlich gemeinsam mit den stickstoffhaltigen Wirkstoffvorstufen. Es ist aber auch möglich, die Quaternierung der Alkylaminalkoxylate allein für sich durchzuführen.

Weiterhin wurde gefunden, daß durch die Mitverwendung der quaternierten Alkylaminalkoxylate die Konzentration der Wirksubstanz in den Fertigformulierungen von bisher maximal 20 Gew.-% deutlich, und zwar auf 30 bis 60 Gew.-% angehoben werden kann.

Als Wirksubstanz werden hier die Mischungen aus den quaternierten stickstoffhaltigen kationenaktiven Wirkstoffkomponenten, die wiederum Mischungen sein können, und den quaternierten Alkylaminalkoxylaten verstanden.

Die Dispersionen enthalten weiterhin pH-Regulatoren, wie z. B. Natronlauge, und Duft- und Farbstoffe. Sie können auch bis zu 2 Gew.-% übliche Salze wie z. B. CaCl₂ oder MgCl₂ enthalten.

Der Zusatz der Viskositätsregulatoren kann sowohl zu den stickstoffhaltigen kationenaktiven Wirkstoffkomponenten allein als auch zu den gesamten Mischungen erfolgen.

Es werden 0,5 bis 50 % (bezogen auf die Wirksubstanz) der quaternierten Alkylaminalkoxylate in Fertigdispersionen, die 15 bis 60 Gew.-%, bevorzugt 20 bis 50 Gew. -%, Wirksubstanz enthalten, verwendet.

Durch die Verwendung der quaternierten Alkylaminalkoxylate kann die Lagerstabilität von stickstoffhaltigen kationenaktiven Wirkstoffkomponenten erfindungsgemäß so eingestellt werden, daß bei Lagerung über einen Temperaturbereich von 0 bis 50 °C keine nachteilige Viskositätsveränderung, insbesondere kein störender Anstieg auftritt. Eine merkliche Eindickung findet nicht statt.

Die nachfolgenden Beispiele sollen die Erfindung verdeutlichen:

| Beispiel | 1 | 2 | 3 | 4 | 5 |
|---|---|---|---|---|---|
| Fettsäureester des N-Methyl-N,N,N-trihydroxyethylammonium-methylsulfats | 20 | 20 | 25 | 25 | 30 |
| Quatern. C_{16/18}-Alkylaminethoxylat (50 E0) | 1 | 3 | 3 | 3 | 4 |
| MgCl₂ | 0,6 | - | 0,6 | - | 0,8 |
| CaCl₂ 10%ige Lösung | - | - | - | 1,2 | - |
| Natronlauge 10%ig | 0,6 | 0,6 | 0,6 | 0,6 | 0,6 |
| Parfüm | 0,5 | 0,5 | 0,5 | 0,5 | 0,5 |
| | Rest zu 100 % Wasser | | | | |

| Beispiel | 6 | 7 | 8 | 9 | 10 | 11 |
|---|---|---|---|---|---|---|
| 1-Methyl-2-alkyl(C₁₆C₁₈)-3-alkyl(C₁₆C₁₈)amidoethylimidazoliniummethosulfat | 20 | 30 | - | - | - | - |
| 2,3-Dihydroxypropyl-1-tri-methylammoniumsalz | - | - | 20 | 28 | - | - |
| N,N-Di(stearyl)-N,N-di(methyl)ammoniumsalz | - | - | - | - | 20 | 25 |
| Quatern. C₁₆-C₁₈-aminethoxylat (50 E0) | 0,8 | 3,5 | 1 | 2,5 | 1 | 2 |
| MgCl₂ x 6 H₂O | 0,5 | - | - | 1 | 0,6 | 1 |
| MgCl₂ 25%ige Lösung | - | 1,4 | 0,6 | - | - | - |
| Natronlauge 10%ig | 0,6 | 0,8 | 0,6 | 0,7 | 0,6 | 0,8 |
| Parfüm | 0,5 | 0,5 | 0,5 | 0,5 | 0,5 | 0,5 |
| | Rest zu 100 % Wasser | | | | | |

Alle Zahlenangaben in der aufgeführten Tabelle bedeuten Gew.-%.

Die Viskositäten liegen nach der Herstellung und 6-wöchiger Lagerung (4 - 40 °C) unter 100 mPa·s, gemessen bei 25 °C.

## Patentansprüche

1. Verwendung von quaternierten Alkylaminalkoxylaten der allgemeinen Formeln
R₁R₃N^{⊕}[(C₂H₄O)ₓ(C₃H₆O)_{y}-H]₂ X^{⊖} (I)
oder
R₁R₂R₃N^{⊕}[(C₂H₄O)ₓ(C₃H₆O)_{y}-H] X^{⊖} (II)
oder deren Mischungen, wobei R₁ und R₂ unabhängig voneinander verzweigte oder geradkettige, gesättigte oder ungesättigte Alkylreste mit 8 bis 22, vorzugsweise 12 bis 18, Kohlenstoffatomen bedeuten, R₃ für einen Alkylrest mit 1 bis 4 Kohlenstoffatomen oder einen Alkylarylrest mit 7 bis 10 Kohlenstoffatomen steht, X^{⊖} einen einwertigen Salzrest darstellt und für x und y
x = 0 bis 150, vorzugsweise 10 bis 100,
y = 0 bis 100, vorzugsweise 0 bis 10 gilt und
x oder y mindestens 1 sein muß
und C₂H₄O eine Ethylenoxid-Moleküleinheit und C₃H₆O eine Propylenoxid-Moleküleinheit bedeutet,
als Viskositätsregulatoren für wäßrige Dispersionen von einzelnen stickstoffhaltigen kationenaktiven Wirkstoffkomponenten oder deren Mischungen und üblichen Zusatzstoffen.

2. Verwendung von 0,5 bis 50 % (bezogen auf die Wirksubstanz) der quaternierten Alkylaminalkoxylate nach Anspruch 1 in Fertigdispersionen, die einzelne stickstoffhaltige kationenaktive Wirkstoffkomponenten oder deren Mischungen und quaternierte Alkylaminalkoxylate als Wirksubstanz enthalten.

3. Verwendung von quaternierten Alkylaminalkoxylaten nach den Ansprüchen 1 und 2 in Fertigdispersionen, die 15 bis 60 Gew.-%, bevorzugt 20 bis 50 Gew.-%, Wirksubstanz bestehend aus einzelnen stickstoffhaltigen kationenaktiven Wirkstoffkomponenten oder deren Mischungen und den quaternierten Alkylaminalkoxylaten enthalten.

4. Verwendung von quaternierten Alkylaminalkoxylaten nach den Ansprüchen 1 bis 3 in Fertigdispersionen, die Fettsäureester der N-Alkyl-N,N,N-trihydroxyalkylammoniumsalze als stickstoffhaltige kationenaktive Wirkstoffkomponenten enthalten.

5. Verwendung von quaternierten Alkylaminalkoxylaten nach den Ansprüchen 1 bis 3 in Fertigdispersionen, die quaternierte bzw. protonierte Imidazoline als stickstoffhaltige kationenaktive Wirkstoffkomponenten enthalten.

6. Verwendung von quaternierten Alkylaminalkoxylaten nach den Ansprüchen 1 bis 3 und 5 in Fertigdispersionen, die 1-Methyl-2-alkyl(C₁₆C₁₈)-3-alkyl(C₁₆C₁₈)-amidoethylimidazoliniummethosulfate als stickstoffhaltige kationenaktive Wirkstoffkomponenten enthalten.

7. Verwendung von quaternierten Alkylaminalkoxylaten nach den Ansprüchen 1 bis 3 in Fertigdispersionen, die 2,3-Dihydroxyalkyl-1-trialkylammoniumsalz-Derivate als stickstoffhaltige kationenaktive Wirkstoffkomponenten enthalten.

8. Verwendung von quaternierten Alkylaminalkoxylaten nach den Ansprüchen 1 bis 3 und 7 in Fertigdispersionen, die 2,3-Dihydroxypropyl-1-trimethylammoniumsalz-Derivate als stickstoffhaltige kationenaktive Wirkstoffkomponenten enthalten.

9. Verwendung von quaternierten Alkylaminalkoxylaten nach den Ansprüchen 1 bis 3 in Fertigdispersionen, die N,N,N,N-Tetraalkylammoniumsalze als stickstoffhaltige kationenaktive Wirkstoffkomponenten enthalten.

10. Verwendung von quaternierten Alkylaminalkoxylaten nach den Ansprüchen 1 bis 3 und 9 in Fertigdispersionen, die N,N-Distearyl-N,N-dimethylammoniumsalze als stickstoffhaltige kationenaktive Wirkstoffkomponenten enthalten.
